# EUROPEAN PATENT APPLICATION

(11) **EP 4 838 179 A1**
(43) Date of publication of application: **11.11.2026**
(21) Application number: 26176957.4
(22) Date of filing: 06.05.2026
(51) Int. Cl.: G01D 5/347, G01P 3/486, G01P 13/04, G01P 21/02

(54) **SLIP RING ENCODER, ENCODING METHOD AND CT DEVICE**

(30) Priority: 07.05.2025 CN 202510582986
(71) Applicant: Tsinghua University, Haidian District, Beijing 100084 (CN); Nuctech Company Limited, TongFang Building Shuangqinglu Haidian District Beijing 100084 (CN)
(72) Inventor: ZHANG, Li, Beijing, 100084 (CN); ZHANG, Jinyu, Beijing, 100084 (CN); TANG, Hu, Beijing, 100084 (CN); LIANG, Jinning, Beijing, 100084 (CN); XIE, Yunpeng, Beijing, 100084 (CN); CHENG, Yi, Beijing, 100084 (CN); Chunlei, LI, Beijing, 100084 (CN); ZHANG, Linlin, Beijing, 100084 (CN); GUO, Jin, Beijing, 100084 (CN); LI, Yue, Beijing, 100084 (CN)
(74) Representative: Isarpatent

(57) **Abstract**

A slip ring encoder, an encoding method and a CT device are provided, which may be applied to a field of encoders and CT devices. The slip ring encoder includes: an encoding strip (1) having a plurality of first-type apertures regularly arranged; a plurality of mutually independent signal acquisition boards (21-23) arranged on one side of the encoding strip (1), where each of the signal acquisition boards (21-23) includes a first signal sensor configured to, when rotating relative to the encoding strip (1), generate a first acquisition signal according to light emitted from the other side of the encoding strip (1) and passing through the plurality of first-type apertures; and a signal processing board (4) electrically connected to the plurality of signal acquisition boards (21-23) and configured to generate a first encoding signal based on at least one of the plurality of first acquisition signals.

## Description

### TECHNICAL FIELD

The present disclosure relates to a field of encoders and CT devices, in particular to a slip ring encoder, an encoding method and a CT device.

### BACKGROUND

A CT (Computed Tomography) device is a medical device that typically uses a precisely collimated X-ray beam, gamma ray, ultrasonic wave, etc., together with a highly sensitive detector to perform successive sectional scans around a certain part of the human body, and generates a three-dimensional image of the part after computer processing. At present, one method used for measuring the rotational speed of a slip ring on similar CT devices is speed measurement by means of a slip ring encoding strip and photoelectric sensors, and another method is speed measurement by means of proximity switches. Speed measurement by a slip ring encoding strip features high real-time performance. When the slip ring runs at a high speed, the signal output time may reach the order of hundreds of microseconds, which may well detect the instantaneous speed of the slip ring.

The existing encoding strip may have an aperture blockage problem. Once aperture blockage occurs on the encoding strip, the rotational speed measurement will be inaccurate, thereby affecting the position information bound to data acquisition, and ultimately affecting images. In the prior art, paired photoelectric sensors are typically arranged on one detection module to serve as redundant detection for each other. The two photoelectric sensors are on the same circuit board. After a plurality of consecutive apertures are blocked due to dust accumulation or grease contamination on the encoding strip, the signals of the two photoelectric sensors will fail simultaneously, which easily leads to the loss of output pulse signals.

### SUMMARY

In view of the above-mentioned problems, the present disclosure provides a slip ring encoder, an encoding method and a CT device that may effectively prevent encoder failure caused by blockage of consecutive apertures. The present disclosure reduces the probability of loss of acquisition signals of the encoding strip caused by local consecutive aperture blockage of the encoding strip by adopting a plurality of signal acquisition boards to perform signal detection on the encoding strip.

According to a first aspect of the present disclosure, there is provided a slip ring encoder, including: an encoding strip having a plurality of first-type apertures regularly arranged; a plurality of mutually independent signal acquisition boards arranged on one side of the encoding strip, where each of the signal acquisition boards includes a first signal sensor configured to, when rotating relative to the encoding strip, generate a first acquisition signal according to light emitted from the other side of the encoding strip and passing through the plurality of first-type apertures; and a signal processing board electrically connected to the plurality of signal acquisition boards and configured to generate a first encoding signal based on at least one of the plurality of first acquisition signals.

According to embodiments of the present disclosure, the plurality of signal acquisition boards are arranged on one side of the encoding strip, and the plurality of signal acquisition boards are configured to generate the plurality of first acquisition signals having a substantially same phase.

According to embodiments of the present disclosure, the plurality of signal acquisition boards are arranged separately from the signal processing board.

According to embodiments of the present disclosure, generating a first encoding signal based on at least one of the plurality of first acquisition signals includes: the signal processing board being configured to select one of the first acquisition signals to generate the first encoding signal, where, in a case that the selected first acquisition signal is lost, another first acquisition signal is selected to generate the first encoding signal.

According to embodiments of the present disclosure, adjacent signal acquisition boards are arranged on one side of the encoding strip at a central angle interval of more than 1°.

According to embodiments of the present disclosure, each of the signal acquisition boards further includes a second signal sensor configured to, when rotating relative to the encoding strip, generate a plurality of second acquisition signals according to light emitted from the other side of the encoding strip and passing through the plurality of first-type apertures; the first acquisition signal and the second acquisition signal generated by each of the signal acquisition boards have a preset phase difference, based on a relative position difference between the first signal sensor, the second signal sensor and any one of the plurality of first-type apertures; the signal processing board is further configured to generate a second encoding signal based on at least one of the plurality of second acquisition signals; and the first encoding signal and the second encoding signal are used to determine a rotation direction of a slip ring.

According to embodiments of the present disclosure, the preset phase difference between the first acquisition signal and the second acquisition signal generated by each of the signal acquisition boards is between 1/4 and 3/4 of a signal period.

According to embodiments of the present disclosure, the encoding strip further has a second-type aperture; each of the signal acquisition boards further includes a third signal sensor configured to, when rotating relative to the encoding strip, generate a plurality of third acquisition signals according to light emitted from the other side of the encoding strip and passing through the second-type aperture; and the signal processing board is further configured to generate a third encoding signal based on at least one of the plurality of third acquisition signals, and the third encoding signal is used to determine a number of rotations of a slip ring.

Another aspect of embodiments of the present disclosure provides an encoding method for a slip ring encoder, applied to the above-mentioned slip ring encoder, where the method includes: when a plurality of first signal sensors of a plurality of signal acquisition boards rotate relative to an encoding strip, generating a plurality of first acquisition signals based on light emitted from the other side of the encoding strip and passing through the plurality of first-type apertures; and generating a first encoding signal based on at least one of the plurality of first acquisition signals.

According to embodiments of the present disclosure, the method further includes: detecting whether the first acquisition signal is lost; and indicating an abnormal position of the encoding strip based on a loss position of the first acquisition signal.

According to embodiments of the present disclosure, the generating a first encoding signal based on at least one of the plurality of first acquisition signals includes: selecting one of the first acquisition signals to generate the first encoding signal, where, in a case that the selected first acquisition signal is lost, another first acquisition signal is selected to generate the first encoding signal.

According to embodiments of the present disclosure, the method further includes: when a plurality of second signal sensors of the plurality of signal acquisition boards rotate relative to the encoding strip, generating a plurality of second acquisition signals according to light emitted from the other side of the encoding strip and passing through the plurality of first-type apertures; generating a second encoding signal based on one of the plurality of second acquisition signals; and determining a rotation direction of a slip ring based on the first encoding signal and the second encoding signal, where each of the signal acquisition boards further includes the second signal sensor, and the first acquisition signal and the second acquisition signal generated by each of the signal acquisition boards have a preset phase difference, based on a relative position difference between the first signal sensor, the second signal sensor and any one of the plurality of first-type apertures.

According to embodiments of the present disclosure, the method further includes: when a plurality of third signal sensors of the plurality of signal acquisition boards rotate relative to the encoding strip, generating a plurality of third acquisition signals according to light emitted from the other side of the encoding strip and passing through a second-type aperture; and generating a third encoding signal based on at least one of the plurality of third acquisition signals, and determining a number of rotations of a slip ring based on the third encoding signal, where the encoding strip has one second-type aperture, and each of the signal acquisition boards further includes the third signal sensor.

According to embodiments of the present disclosure, the method further includes: accumulating the first encoding signal, and incrementing the number of rotations of the slip ring by one based on the third encoding signal and an accumulated count value of the first encoding signal reaching a preset value.

Another aspect of embodiments of the present disclosure provides a CT device, including the slip ring encoder described above.

One or more of the above-mentioned embodiments have the following beneficial effects.

In embodiments of the present disclosure, a plurality of mutually independent signal acquisition boards are provided, and the signal processing board is electrically connected to the plurality of signal acquisition boards to generate a first encoding signal based on at least one of the plurality of first acquisition signals. The limitation in the encoder field that two photoelectric sensors are on the same circuit board and sensors are designed in pairs in the signal acquisition redundancy technology is broken through. When the consecutive aperture blockage area of the encoding strip is large, the encoder may work normally as long as one signal acquisition board may normally generate the first encoding signal at any given moment. The risk of simultaneous failure of encoded signals caused by consecutive aperture blockage of the encoding strip may be mitigated, the probability of loss of trigger signals of the encoding strip caused by local consecutive aperture blockage of the encoding strip is reduced, and the maintenance period of the encoding strip and the photoelectric detection module is prolonged.

In embodiments of the present disclosure, whether the corresponding first acquisition signal is lost is detected based on phase deviation. The fault position of the encoding strip may be conveniently identified, an alarm signal of an aperture blockage region may be given, and blind troubleshooting of the aperture blockage position of the encoding strip may be avoided.

In some embodiments of the present disclosure, each signal acquisition board acquires a first acquisition signal, a second acquisition signal and a third acquisition signal, i.e., A-phase acquisition signal, B-phase acquisition signal and Z-phase acquisition signal. The signal processing board respectively generates a first encoding signal, a second encoding signal and a third encoding signal according to the A/B/Z-phase acquisition signals of each signal acquisition board, and after processing, the accurate rotational speed, rotation direction and number of rotations of the slip ring are finally determined.

In embodiments of the present disclosure, each acquisition module does not require paired signal sensors, such that the failure probability caused by aperture blockage is reduced, the design complexity of the acquisition modules is reduced, the design limitations are reduced, and the cost of a single acquisition module is reduced.

### BRIEF DESCRIPTION OF THE DRAWINGS

The above-mentioned content, as well as other objectives, features and advantages of the present disclosure will become more apparent by describing embodiments of the present disclosure with reference to the accompanying drawings, in which:
FIG. 1 schematically shows a working principle diagram of a slip ring encoder.
FIG. 2 schematically shows a speed measurement principle diagram of an existing slip ring encoder.
FIG. 3 schematically shows a slip ring encoder according to an embodiment of the present disclosure.
FIG. 4 schematically shows a schematic diagram of signal acquisition of a slip ring encoder according to an embodiment of the present disclosure.
FIG. 5 schematically shows another slip ring encoder according to an embodiment of the present disclosure.
FIG. 6 schematically shows another slip ring encoder according to an embodiment of the present disclosure.
FIG. 7 schematically shows a flowchart of an encoding method for a slip ring encoder according to an embodiment of the present disclosure.
FIG. 8 schematically shows a flowchart of another encoding method for a slip ring encoder according to an embodiment of the present disclosure.
FIG. 9 schematically shows a flowchart of another encoding method for a slip ring encoder according to an embodiment of the present disclosure.
FIG. 10 schematically shows a structure diagram of a CT device according to an embodiment of the present disclosure.
FIG. 11 schematically shows a partial structure diagram of a CT device according to an embodiment of the present disclosure.

In the accompanying drawings: 1, Encoding strip; 2, 21-23, Signal acquisition board; 3, 31-33, Light-emitting device; 4, Signal processing board; 5, Slip ring; 6, CT device body.

It should be noted that, for the sake of clarity, dimensions of overall/local structures or overall/local regions in the accompanying drawings used to describe embodiments of the present disclosure may be exaggerated or reduced, i.e., the accompanying drawings are not drawn to an actual scale.

### DETAILED DESCRIPTION OF EMBODIMENTS

Embodiments of the present disclosure will be described below with reference to the accompanying drawings. It should be understood, however, that these descriptions are merely exemplary and are not intended to limit the scope of the present disclosure. In the following detailed descriptions, for purposes of explanation, numerous specific details are set forth in order to provide a thorough understanding of embodiments of the present disclosure. It is obvious, however, that one or more embodiments may be implemented without these specific details. In addition, in the following descriptions, descriptions of well-known structures and technologies are omitted to avoid unnecessarily obscuring the concept of the present disclosure.

Terms used herein are for the purpose of describing embodiments only and are not intended to limit the present disclosure. Terms "comprising", "including" and the like used herein specify a presence of the feature, step, operation and/or component, but do not preclude a presence or addition of one or more other features, steps, operations or components.

All terms (including technical and scientific terms) used herein have the meaning as commonly understood by those skilled in the art, unless otherwise defined. It should be noted that the terms used herein should be construed to have meanings consistent with the context of the present description and should not be construed in an idealized or overly rigid manner.

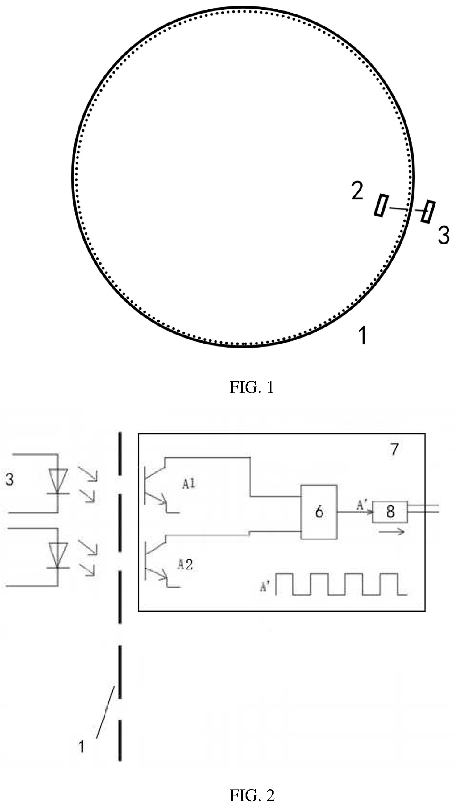

FIG. 1 shows a working principle diagram of an existing slip ring encoder. As shown in FIG. 1 and FIG. 2, the slip ring encoder includes an encoding strip 1 and a signal acquisition board 2 arranged on one side of the encoding strip. The signal acquisition board 2 may adopt an optical signal acquisition technology, for example, the signal acquisition board includes a photoelectric sensor. The encoding strip may be a steel strip. The encoding strip is fixed to the slip ring and may rotate together with the slip ring. The encoding strip 1 is provided with regularly arranged apertures. A light-emitting device 3 (e.g., a light-emitting diode) is arranged on the other side of the encoding strip 1 opposite to the signal acquisition board 2. The signal acquisition board 2 and the light-emitting device 3 remain relatively stationary. Light emitted by the light-emitting device 3 passes through the apertures in the encoding strip and may be acquired by the signal acquisition board 2. The signal acquisition board 2 converts the acquired optical signals into electrical signals with high or low levels. In this way, when the encoding strip 1 and the signal acquisition board 2 rotate relative to each other, the light beam is alternately transmitted and blocked, and the sensed optical signals may be converted into electrical signals or pulses related to the rotational speed, with the pulse frequency being proportional to the rotational speed of the slip ring.

During long-term operation, the apertures of the encoding strip 1 in the slip ring encoder are often completely or partially blocked due to foreign matter such as dust. Since the slip ring encoding strip speed measurement technology has high requirements for signals, if redundant sensors are configured for the sensor, the mutually redundant sensors must have the same phase. Otherwise, the speed measurement and the measurement of other parameters will be affected.

In the art, in order to prevent aperture blockage of the encoding strip, technicians have long had to arrange two sensors in pairs on the same sensor circuit board. When processing the sensor circuit board, the positions of the two sensors may be predetermined, and the positions of the two sensors are related to the aperture positions of the encoding strip. The paired design and fixed positions of the sensors ensure that the mutually redundant sensors have the same phase. As shown in FIG. 2, a pair of sensors is provided on one circuit board 7 to respectively generate two sensing signals A1 and A2, and a light-emitting device 3 is provided at a position opposite to the sensors, where the light-emitting device 3 is separated from the sensors by the encoding strip 1. The distance between the pair of sensors is fixed in advance, and the two sensing signals A1 and A2 generated after receiving light emitted by the light-emitting device 3 are in the same phase. A signal combination module 6 is further provided on the circuit board 7, and is used to perform an OR operation on the sensing signals A1 and A2 to generate an A' signal. The A' signal is processed by a differential module 8 and then output externally.

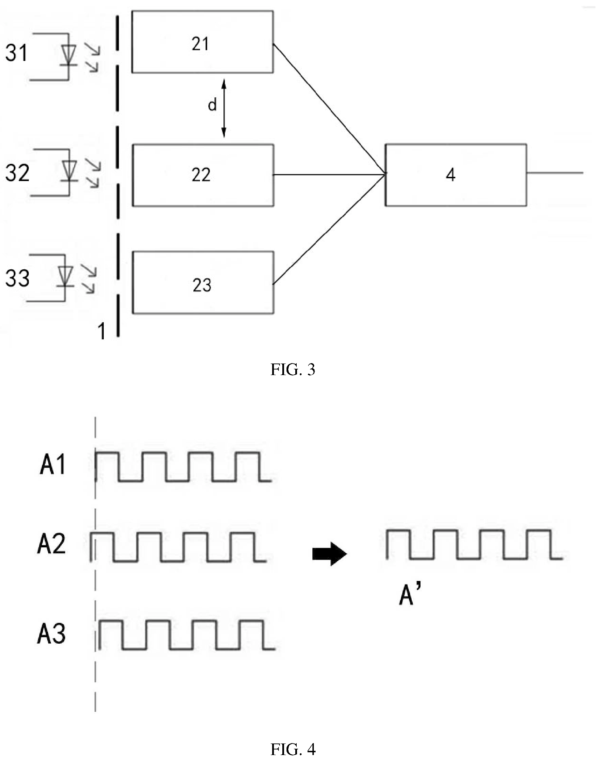

The slip ring encoder according to embodiments of the present disclosure will be described in detail below with reference to FIG. 3 to FIG. 5.

FIG. 3 schematically shows a slip ring encoder according to an embodiment of the present disclosure.

As shown in FIG. 3, the embodiment includes:
an encoding strip 1 having a plurality of first-type apertures regularly arranged; a plurality of mutually independent signal acquisition boards 21-23 arranged on one side of the encoding strip, where each signal acquisition board includes one first signal sensor configured to, when rotating relative to the encoding strip, generate a first acquisition signal according to light emitted from the other side of the encoding strip and passing through the plurality of first-type apertures; and a signal processing board 4 electrically connected to the plurality of signal acquisition boards and configured to generate a first encoding signal based on at least one of the plurality of first acquisition signals. The first encoding signal is used to determine the rotational speed of the slip ring.

In embodiments of the present disclosure, the signal acquisition board is a circuit board including a photoelectric sensor, which may use the photoelectric effect: when light irradiates a photodiode, a photocurrent is formed to achieve optical signal acquisition. In embodiments of the present disclosure, the plurality of signal acquisition boards are manufactured, arranged, and perform signal acquisition independently of each other. The first signal sensor of each signal acquisition board independently generates one first acquisition signal during operation, such that the photoelectric sensors of different signal acquisition boards are not restricted by phase or distance during the manufacturing process. Second, the plurality of signal acquisition boards are independently installed, and the plurality of signal acquisition boards are not arranged on the same circuit board, and may be arranged adjacently or dispersedly on one side of the encoding strip. Each signal acquisition board independently completes signal acquisition by means of its own photoelectric sensor, and the acquired first acquisition signal is further processed by the signal processing board. In embodiments of the present disclosure, the signal processing board may generally be provided independently of the signal acquisition boards, or may be provided on one of the signal acquisition boards. Preferably in the present embodiment, the plurality of signal acquisition boards are arranged separately from the signal processing board. By separating the signal acquisition boards of the slip ring encoding strip from the signal processing board and adopting the plurality of slip ring encoding strip signal acquisition boards to detect encoding strip signals, the plurality of slip ring encoding strip signal acquisition boards are distributed at intervals on the slip ring, and the probability of loss of trigger signals of the encoding strip caused by local consecutive aperture blockage of the encoding strip is reduced.

In the present embodiment, in an operating state, relative rotation occurs between the signal acquisition boards and the encoding strip. Specifically, the signal acquisition boards may be fixed to the slip ring and rotate together with the slip ring, while the encoding strip is fixed to the device and does not rotate with the slip ring. Alternatively, the encoding strip may be fixed to the slip ring and rotate together with the slip ring.

In the present embodiment, each signal acquisition board includes one first signal sensor configured to generate a first acquisition signal according to light emitted from the other side of the encoding strip and passing through the plurality of first-type apertures when rotating relative to the encoding strip. In a normal operating state, the first signal sensors included in the signal acquisition boards 21-23 are configured to generate a plurality of first acquisition signals according to light emitted from the other side of the encoding strip and passing through the plurality of first-type apertures when rotating relative to the encoding strip. For example, the first signal sensor of the signal acquisition board 21 receives light emitted by the light-emitting device 31 on the other side of the encoding strip 1 and passing through the apertures of the encoding strip 1 to generate a first acquisition signal A1; the first signal sensor of the signal acquisition board 22 receives light emitted by the light-emitting device 32 on the other side of the encoding strip 1 and passing through the apertures of the encoding strip 1 to generate a first acquisition signal A2; the first signal sensor of the signal acquisition board 23 receives light emitted by the light-emitting device 33 on the other side of the encoding strip 1 and passing through the apertures of the encoding strip 1 to generate a first acquisition signal A3. It should be noted that the first acquisition signals A1, A2 and A3 are generated simultaneously in the operating state. The signal processing board 4 is connected to the signal acquisition boards and configured to generate a first encoding signal A' based on at least one of the plurality of first acquisition signals A1, A2 and A3. In a case that the encoding strip has local aperture blockage, at a certain moment, when the signal acquisition board 21 rotates to an aperture-blocked position of the encoding strip, the signal acquisition board 21 may not generate a first acquisition signal, while the signal acquisition boards 22 and 23 normally generate the first acquisition signal A2 and the first acquisition signal A3. At another moment, the signal acquisition board 21 leaves the aperture-blocked position of the encoding strip and the signal acquisition board 22 rotates to the aperture-blocked position of the encoding strip, and the signal acquisition board 22 may not generate a first acquisition signal, while the signal acquisition boards 21 and 23 normally generate the first acquisition signal A1 and the first acquisition signal A3. At yet another moment, the signal acquisition board 22 leaves the aperture-blocked position of the encoding strip and the signal acquisition board 23 rotates to the aperture-blocked position of the encoding strip, and the signal acquisition board 23 may not generate a first acquisition signal, while the signal acquisition boards 21 and 22 normally generate the first acquisition signal A1 and the first acquisition signal A2. Therefore, in a case that the encoding strip has local aperture blockage, although different signal acquisition boards alternately lose position encoding information, other signal acquisition boards may still work normally. When the consecutive aperture blockage area of the encoding strip is large, normal operation may be maintained as long as at least one signal acquisition board is functioning normally at any given moment to generate the first encoding signal. Generating a first encoding signal based on at least one of the plurality of first acquisition signals may be performing an OR operation on the plurality of first acquisition signals to generate the first encoding signal, or selecting one of the signals as the first encoding signal, and when the selected first acquisition signal is lost, selecting another first acquisition signal as the first encoding signal. The present embodiment preferably adopts the latter manner, which will be described in further detail in subsequent embodiments.

By adopting a design concept different from that in the prior art, the present embodiment uses a plurality of signal acquisition boards to perform signal detection on the encoding strip, redundant sensors do not need to be designed in each signal acquisition board, simultaneous failure of encoding signals caused by consecutive aperture blockage of the encoding strip may be avoided to a greater extent, and the probability of loss of trigger signals of the encoding strip caused by local consecutive aperture blockage of the encoding strip is reduced.

In some embodiments of the present disclosure, the plurality of signal acquisition boards are arranged on one side of the encoding strip, and the plurality of signal acquisition boards are configured to generate the plurality of first acquisition signals having a substantially same phase. In the general understanding in the art, only signals of the same phase may be used as one signal. In the present embodiment, the plurality of signal acquisition boards are arranged on one side of the encoding strip, and the plurality of signal acquisition boards are configured to generate the plurality of first acquisition signals having a substantially same phase, which greatly reduces the implementation difficulty, such that the plurality of signal acquisition boards 21-23 do not have to be processed on the same circuit board and maintain a preset fixed distance to obtain the same signal phase. In the present embodiment, it is sufficient that: the plurality of signal acquisition boards are arranged on one side of the encoding strip, and the plurality of signal acquisition boards are configured to generate the plurality of first acquisition signals having a substantially same phase.

In the present embodiment, when a plurality of slip ring encoding strip signal acquisition boards are mounted on the slip ring for the first time, the phase edges of signal acquisition boards need to be aligned. Generally, for example, alignment is achieved when different signal acquisition boards are positioned such that different signal acquisition boards receive light from the light-emitting devices exactly through the apertures of the encoding strip. During phase adjustment, the signal processing board connected to the signal acquisition boards will give a phase alignment indication, and an indicator light on the signal processing board will light up after the positions of the plurality of signal acquisition boards meet the requirements. Since the signal acquisition boards are later mounted on the slip ring, although the indicator light on the signal processing board lights up, the plurality of first acquisition signals generated by the signal acquisition boards may still have phase deviations. That is, the plurality of signal acquisition boards generate a plurality of first acquisition signals having substantially the same phase.

In some embodiments of the present disclosure, generating a first encoding signal based on at least one of the plurality of first acquisition signals includes: the signal processing board being configured to select one of the first acquisition signals to generate the first encoding signal; where, in a case that the selected first acquisition signal is lost, another first acquisition signal is selected to generate the first encoding signal.

Since in the present embodiment, the plurality of signal acquisition boards are arranged on one side of the encoding strip, and the plurality of signal acquisition boards are configured to generate the plurality of first acquisition signals having a substantially same phase, the first acquisition signals generated by different signal acquisition boards may still have a certain phase deviation. Directly performing an OR operation on different first acquisition signals to generate a first encoding signal has certain disadvantages; especially when these phase deviations reach a certain level, performing an OR operation on different first acquisition signals to generate a first encoding signal does not meet the use conditions, and the duty cycle may even reach 100%. Although the phases of different signal acquisition boards are aligned during mounting of the signal acquisition boards, mechanical vibration, signals, rotational speed and other factors during use may cause the phase deviations of different signal acquisition boards to gradually expand, and the duty cycle of the first encoding signal generated by performing an OR operation on different first acquisition signals may reach 100%, eventually making the first encoding signal generated by performing an OR operation on different first acquisition signals unusable. The present embodiment adopts a manner of monitoring the first acquisition signals generated by the plurality of signal acquisition boards, selecting one of the first acquisition signals to generate the first encoding signal, and when the selected first acquisition signal is lost, selecting another first acquisition signal to generate the first encoding signal. Even if the first acquisition signals of the plurality of signal acquisition boards have a large phase deviation, since only one first acquisition signal is selected to follow under normal conditions, the output waveform is normal; if aperture blockage occurs and another first acquisition signal is selected to follow to generate the first encoding signal, the waveform will only be distorted when switching to follow another first acquisition signal, and a signal duty cycle of 100% or other situations affecting signal reading will not occur.

As shown in FIG. 4, the signal processing board 4 is configured to monitor the first acquisition signals A1, A2 and A3 generated by the signal acquisition boards 21-23, and select the first acquisition signal A1 to generate the first encoding signal A'. When the signal acquisition board 21 moves to an aperture-blocked position of the encoding strip, the selected first acquisition signal A1 is lost at a timing when a pulse signal is expected to be generated, the signal processing board 4 selects the first acquisition signal A2 generated by the signal acquisition board 22 to continue generating the first encoding signal A'. Similarly, when the signal acquisition board 22 moves to an aperture-blocked position of the encoding strip, the selected first acquisition signal A2 is lost at a timing when a pulse signal is expected to be generated, the signal processing board 4 again switches to select the first acquisition signal A3 of the signal acquisition board 23 to continue generating the first encoding signal A'. Since the pulse frequency of the first encoding signal A' is proportional to the rotational speed of the slip ring, the corresponding rotational speed of the slip ring may be further obtained according to the first encoding signal A'. For example, the encoding strip has a total of n apertures, and the slip ring generates n pulses per revolution. According to a slip ring rotational speed of r revolutions per minute, r*n/60 pulses are generated per second, and the signal period = 1/f = 60/(r*n). The real-time rotational speed of the slip ring may be obtained according to the pulse frequency.

In some embodiments, adjacent signal acquisition boards are arranged on one side of the encoding strip at a central angle interval of more than 1°. Since the encoding strip typically has thousands of regularly arranged apertures, for example, the central angle between adjacent apertures on some encoding strips is 0.25°. Adjacent signal acquisition boards are arranged on one side of the encoding strip at a central angle interval of more than 1°. Theoretically, the encoder may work normally as long as the consecutive aperture-blocked region is less than a certain design angle w (where w is 360° minus the largest one of the interval angles between adjacent signal acquisition boards). In an embodiment, for example, when three signal acquisition boards are provided for the slip ring encoder, adjacent signal acquisition boards may be arranged at a central angle interval of more than 10°, and the central angle intervals between the three signal acquisition boards are 10°, 175° and 175° respectively. In this case, the largest one of the interval angles between adjacent signal acquisition boards is 175°, and the signal acquisition boards may work normally as long as the consecutive aperture blockage is less than 185° (185° = 360° - 175°).

Since different interval distances correspond to different circumferential included angles, for convenience of description, the present embodiment may be described in a manner of being separated by a certain distance, which is essentially equivalent. The distance mentioned in the present embodiment refers to a distance along the arc of the encoding strip, not a straight-line distance. As shown in FIG. 3, in the present embodiment, since the encoding strip is annular, the plurality of signal acquisition boards 21-23 are distributed along the circumference of the annular encoding strip, and a distance d exists between two adjacent signal acquisition boards. The distance d may be twice the distance between adjacent apertures of the encoding strip, which corresponds to preventing two consecutive adjacent apertures of the encoding strip from being blocked. Obviously, the distance d may be a value greater than twice the distance between adjacent apertures of the encoding strip. Generally, the greater the distance d, the better the effect of preventing consecutive aperture blockage. Meanwhile, the distances d between adjacent signal acquisition boards may also be different. For example, the distance between the signal acquisition board 21 and the signal acquisition board 22 is d1, the distance between the signal acquisition board 22 and the signal acquisition board 23 is d2, the distance between the signal acquisition board 21 and the signal acquisition board 23 is d3, and the distances d1, d2 and d3 may be the same or different from each other. In practical applications, the distribution of the signal acquisition boards 21, 22 and 23 may be flexibly designed according to a specific use environment, which reduces design restrictions. The arrangement of adjacent signal acquisition boards separated by a certain distance d may also be implemented according to the arrangement of adjacent signal acquisition boards separated by a certain circumferential included angle.

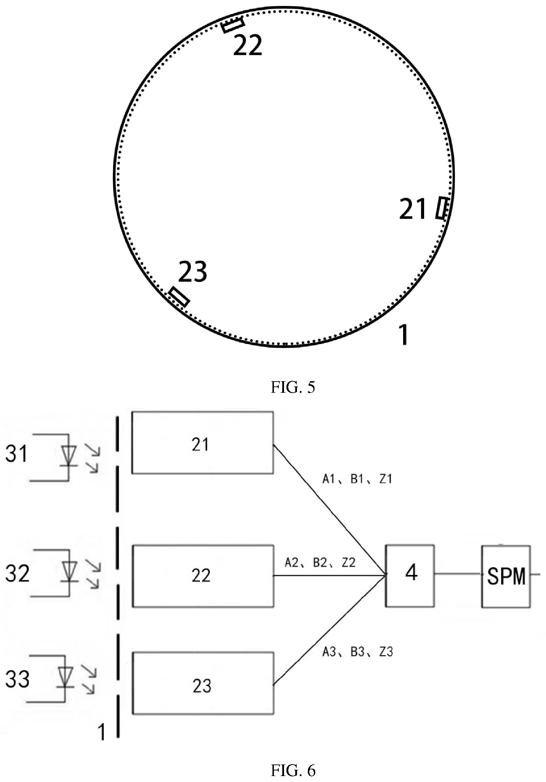

FIG. 5 schematically shows another slip ring encoder according to an embodiment of the present disclosure.

In some embodiments of the present disclosure, N signal acquisition boards are provided, and adjacent signal acquisition boards are arranged on one side of the encoding strip at a central angle interval of 360°/N, where N is an integer greater than or equal to 2. As shown in FIG. 5, in an embodiment of the present disclosure, three signal acquisition boards are provided, adjacent signal acquisition boards are arranged on one side of the encoding strip at a central angle interval of 120°, and the largest one of the interval angles between adjacent signal acquisition boards is 120°. In the present embodiment, even if aperture blockage occurs at a plurality of consecutive positions, correct pulse signal output may be ensured as long as the consecutive aperture blockage is less than 240° (240° = 360° - 120°).

In some embodiments of the present disclosure, each signal acquisition board includes one second signal sensor configured to generate a plurality of second acquisition signals according to light emitted from the other side of the encoding strip and passing through the plurality of first-type apertures when rotating relative to the encoding strip; the first acquisition signal and the second acquisition signal generated by each signal acquisition board have a preset phase difference based on a relative position difference between the first signal sensor, the second signal sensor and any one of the plurality of first-type apertures; the signal processing board is configured to generate a second encoding signal based on at least one of the plurality of second acquisition signals; and the first encoding signal and the second encoding signal are used to determine a rotation direction of a slip ring. In the present embodiment, two photoelectric sensors may be provided on the same signal acquisition board to respectively generate the first acquisition signal and the second acquisition signal. The distance interval between the two photoelectric sensors may be preset, such that the generated first acquisition signal and second acquisition signal have a preset phase difference. Due to the position difference between any one of the plurality of first-type apertures on the encoder and the first signal sensor and the second signal sensor, the time at which any one of the plurality of first-type apertures on the encoder rotates to positions corresponding to the first signal sensor and the second signal sensor differs, thus the generated first acquisition signal and second acquisition signal have a preset phase difference. In the present embodiment, the signal processing board is configured to generate a second encoding signal based on at least one of the plurality of second acquisition signals, which may be specifically: the signal processing board monitors at least one of the second acquisition signals generated by the plurality of signal acquisition boards to generate the second encoding signal, and when the selected second acquisition signal is lost, another second acquisition signal is selected to generate the second encoding signal. The rotation direction of the relative rotation between the signal acquisition board and the encoding strip may be determined through the first encoding signal and the second encoding signal having a preset phase difference, that is, the rotation direction is determined by combining the sequence of occurrence of the first encoding signal and the second encoding signal and the phase difference.

In some embodiments of the present disclosure, the preset phase difference between the first acquisition signal and the second acquisition signal generated by each signal acquisition board is between 1/4 and 3/4 of a signal period. For example, the preset phase difference between the first acquisition signal and the second acquisition signal is 1/4 of a signal period. When the acquisition module and the encoding strip rotate in a first direction, the first acquisition signal leads the second acquisition signal by 1/4 of a signal period; when the acquisition module and the encoding strip rotate in a second direction, the second acquisition signal leads the first acquisition signal by 1/4 of a signal period. For example, the preset phase difference between the first acquisition signal and the second acquisition signal is 3/4 of a signal period. When the acquisition module and the encoding strip rotate in the first direction, the first acquisition signal leads the second acquisition signal by 3/4 of a signal period; when the acquisition module and the encoding strip rotate in the second direction, the second acquisition signal leads the first acquisition signal by 3/4 of a signal period.

In some embodiments of the present disclosure, the encoding strip has one second-type aperture, each signal acquisition board includes one third signal sensor, and each third signal sensor is configured to generate a plurality of third acquisition signals according to light emitted from the other side of the encoding strip and passing through the second-type aperture when rotating relative to the encoding strip; the signal processing board is configured to generate a third encoding signal based on at least one of the plurality of third acquisition signals; and the third encoding signal is used to determine the number of rotations of the slip ring. In the present embodiment, the second-type aperture arranged on each signal acquisition board is dedicated to generating the third acquisition signal. Each time the slip ring rotates one revolution, each third signal sensor generates one third acquisition signal according to light emitted from the other side of the encoding strip and passing through the second-type aperture. Specifically, the second-type aperture is provided on a circumference of the encoding strip, and a radius of the circumference for the second-type aperture is different from a radius of a circumference for the plurality of regularly arranged first-type apertures. The signal processing board may be configured to select one of the third acquisition signals to generate the third encoding signal, where, when the selected third acquisition signal is lost, another third acquisition signal is selected to generate the third encoding signal.

As shown in FIG. 6, in the present embodiment, each signal acquisition board includes a first signal sensor, a second signal sensor and a third signal sensor. When the signal acquisition board and the encoding strip rotate relative to each other, the first signal sensor and the second signal sensor on each signal acquisition board respectively and independently generate a first acquisition signal and a second acquisition signal according to light emitted from a light-emitting device on the other side of the encoding strip and passing through a plurality of different first-type apertures. The generated first acquisition signal and second acquisition signal have a preset phase difference because the apertures corresponding to the two signal sensors are different. The third signal sensor on each signal acquisition board generates a third acquisition signal according to light emitted from the light-emitting device on the other side of the encoding strip and passing through the second-type aperture. Specifically, the slip ring encoder includes a plurality of signal acquisition boards 21-23. The signal acquisition board 21 is configured to, when rotating relative to the encoding strip, generate a first acquisition signal A1, a second acquisition signal B1 and a third acquisition signal Z1 respectively by means of the three signal sensors of the signal acquisition board according to light emitted from the light-emitting device 31 on the other side of the encoding strip and passing through the plurality of first-type apertures. The signal acquisition board 22 is configured to, when rotating relative to the encoding strip, generate a first acquisition signal A2, a second acquisition signal B2 and a third acquisition signal Z2 according to light emitted from the light-emitting device 32 on the other side of the encoding strip and passing through the apertures of the encoding strip. The signal acquisition board 23 is configured to, when rotating relative to the encoding strip, generate a first acquisition signal A3, a second acquisition signal B3 and a third acquisition signal Z3 according to light emitted from the light-emitting device 33 on the other side of the encoding strip and passing through the apertures of the encoding strip. The signal processing board 4 is connected to the signal acquisition boards 21-23 and configured to generate a first encoding signal A' based on at least one of the plurality of first acquisition signals A1-A3, generate a second encoding signal B' based on at least one of the plurality of second acquisition signals B1-B3, and generate a third encoding signal Z' based on at least one of the plurality of third acquisition signals Z1-Z3. The first encoding signal A' is used to determine the rotational speed of the slip ring; the first encoding signal A' and the second encoding signal B' have a phase difference therebetween, which is used to determine the rotation direction of the slip ring; the third encoding signal Z' is used to determine the number of rotations of the slip ring. The first encoding signal A', the second encoding signal B' and the third encoding signal Z' processed by the processing module 4 are transmitted to a data acquisition module SPM.

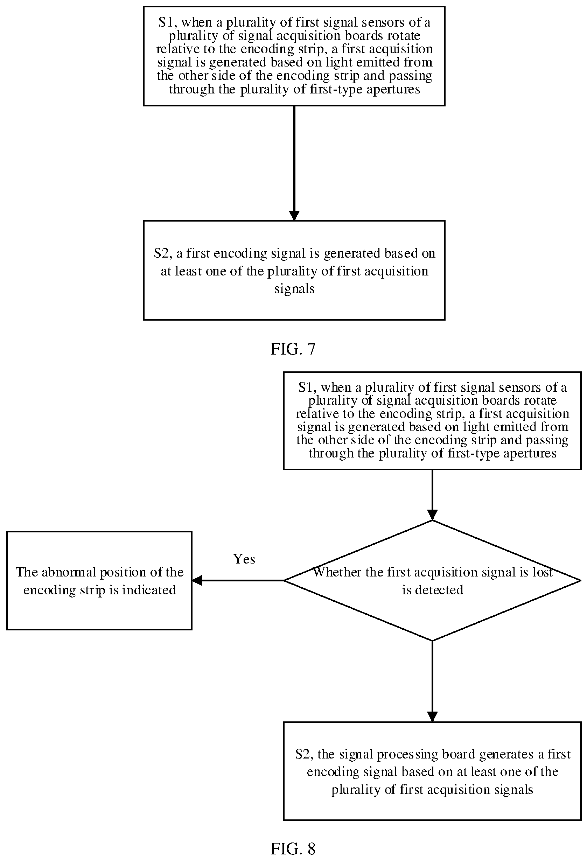

Based on the above-mentioned slip ring encoder, the present disclosure further provides an encoding method for a slip ring encoder. The method will be described in detail below with reference to FIG. 7 to FIG. 9.

FIG. 7 schematically shows a flowchart of an encoding method for a slip ring encoder according to an embodiment of the present disclosure.

As shown in FIG. 7, the encoding method for a slip ring encoder according to the present embodiment includes the following steps: S1, when a plurality of first signal sensors of a plurality of signal acquisition boards rotate relative to the encoding strip, the first signal sensor of each signal acquisition board generates a first acquisition signal based on light emitted from the other side of the encoding strip and passing through the plurality of first-type apertures; S2, a first encoding signal is generated based on at least one of the plurality of first acquisition signals.

In step S2, the generated first encoding signal may be finally used to determine the rotational speed of the slip ring after processing. The working principle of the present embodiment has been described in detail in other above-mentioned embodiments and will not be described in detail here. In the present embodiment, if the encoding strip has local aperture blockage, different signal acquisition boards will alternately lose position encoding information. Due to the existence of the plurality of signal acquisition boards, the problem of encoding signals failing simultaneously due to consecutive aperture blockage of the encoding strip may be avoided, the probability of loss of trigger signals of the encoding strip caused by local consecutive aperture blockage of the encoding strip is reduced, and the maintenance period of the encoding strip and the signal acquisition boards is prolonged.

FIG. 8 schematically shows a flowchart of another encoding method for a slip ring encoder according to an embodiment of the present disclosure.

In some embodiments of the present disclosure, the method further includes: detecting whether the first acquisition signal is lost, and indicating an abnormal position of the encoding strip based on a loss position of the first acquisition signal.

As shown in FIG. 8, in the present embodiment, after operation S1 (i.e., when a plurality of first signal sensors of a plurality of signal acquisition boards rotate relative to the encoding strip, first acquisition signals are generated based on light emitted from the other side of the encoding strip and passing through the plurality of first-type apertures), the method includes a step of detecting whether the first acquisition signal is lost. If a lost first acquisition signal exists, the position of the lost first acquisition signal is the abnormal position of the encoding strip, and the abnormal position of the encoding strip is indicated. Step S2 (i.e., the signal processing board generates a first encoding signal based on at least one of the plurality of first acquisition signals) is then performed. In a specific implementation, if the encoding strip has local aperture blockage, different signal acquisition boards alternately lose position encoding information when rotating to the aperture-blocked position; if a signal acquisition board is faulty, this signal acquisition board will consistently fail to provide pulse signals, allowing the type of fault to be identified for alarming.

In some embodiments of the present disclosure, the generating a first encoding signal based on at least one of the plurality of first acquisition signals includes selecting one of the first acquisition signals to generate the first encoding signal. When the selected first acquisition signal is lost, another first acquisition signal is selected to generate the first encoding signal. For the related principle, please refer to FIG. 4 and related embodiments.

In some embodiments of the present disclosure, the method further includes: when a plurality of second signal sensors of a plurality of signal acquisition boards rotate relative to the encoding strip, generating a plurality of second acquisition signals according to light emitted from the other side of the encoding strip and passing through the plurality of first-type apertures.

The first acquisition signal and the second acquisition signal generated by each signal acquisition board have a preset phase difference. A second encoding signal is generated based on at least one of the plurality of second acquisition signals. A rotation direction of the slip ring is determined based on the first encoding signal and the second encoding signal. Each signal acquisition board includes one second signal sensor. Based on a relative position difference between the first signal sensor, the second signal sensor and any one of the plurality of first-type apertures, the first acquisition signal and the second acquisition signal generated by each signal acquisition board have a preset phase difference.

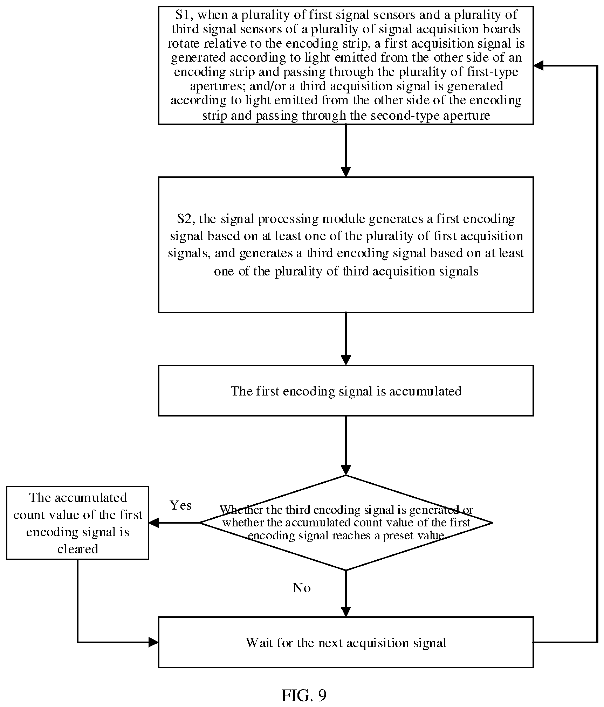

FIG. 9 schematically shows a flowchart of another encoding method for a slip ring encoder according to an embodiment of the present disclosure.

In some embodiments of the present disclosure, the method includes: when a plurality of first signal sensors and a plurality of third signal sensors of a plurality of signal acquisition boards rotate relative to the encoding strip, generating, by the plurality of first signal sensors, a plurality of first acquisition signals according to light emitted from the other side of an encoding strip and passing through the plurality of first-type apertures; and/or generating, by the plurality of third signal sensors, a plurality of third acquisition signals according to light emitted from the other side of the encoding strip and passing through the second-type aperture; generating a third encoding signal based on at least one of the plurality of third acquisition signals; and determining the number of rotations of the slip ring based on the third encoding signal. The encoding strip has one second-type aperture, and each signal acquisition board includes one third signal sensor.

In some embodiments of the present disclosure, determining the number of rotations of the slip ring based on the third encoding signal includes: generating a first encoding signal based on at least one of the plurality of first acquisition signals, accumulating the first encoding signal, and incrementing the number of rotations of the slip ring by one based on the third encoding signal and an accumulated count value of the first encoding signal reaching a preset value. For example, in the present embodiment, the slip ring may generate a preset number of first encoding signals per revolution. When the third encoding signal may not be generated normally for various reasons, the accumulated value of the first encoding signals reaching the preset value may be used to determine that the number of rotations of the slip ring is incremented by one. If the third encoding signal is normal, mutual verification may be performed by means of the accumulated value of the first encoding signals. For example, when one third encoding signal is generated, the accumulated value of the first encoding signals should also reach the preset value; at this point, the accumulated value of the first encoding signals is cleared and accumulation is restarted. Meanwhile, signal loss may occur during transmission of the first encoding signal and the third encoding signal to the data acquisition module. By designing mutual verification between the two, the present embodiment may further improve the encoding strip fault detection capability and facilitate timely detection of whether a fault exists. In some embodiments of the present disclosure, the second encoding signals may also be accumulated for mutual verification with the third encoding signal, which is essentially the same and will not be described in detail.

As shown in FIG. 9, in some embodiments of the present disclosure, the first encoding signal is used for accumulation counting, and the number of rotations of the slip ring is incremented by one based on the third encoding signal and the accumulated count value of the first encoding signal reaching a preset value. In the present embodiment, in step S1, when the encoding strip and the signal acquisition boards rotate relative to each other, each signal acquisition board generates a first acquisition signal and/or a third acquisition signal according to light emitted from the other side of the encoding strip and passing through the plurality of first-type apertures. In step S2, the signal processing board generates a first encoding signal based on at least one of the plurality of first acquisition signals, and generates a third encoding signal based on at least one of the plurality of third acquisition signals. After step S2, the method includes: accumulating the first encoding signal, and determining whether the accumulated value of the first encoding signal reaches a preset value and whether the third encoding signal is generated. When the accumulated value of the first encoding signal reaches the preset value or the third encoding signal is generated, the accumulated count value of the first encoding signal is cleared and the number of rotations of the slip ring is incremented by one. When the accumulated value of the first encoding signal does not reach the preset value, awaiting the next acquisition signal. For example, in the present embodiment, the encoding strip has a total of 1440 first-type apertures, and it is determined whether the accumulated value of the first encoding signals reaches 1440. When the first encoding signals are accumulated to 1440, and the third encoding signal is also generated at this point, the number of rotations of the slip ring is incremented by one. When the first encoding signals are accumulated to 1440 but the third encoding signal is not generated, there may be an abnormality in the generation process of the third encoding signal, and troubleshooting may be prompted.

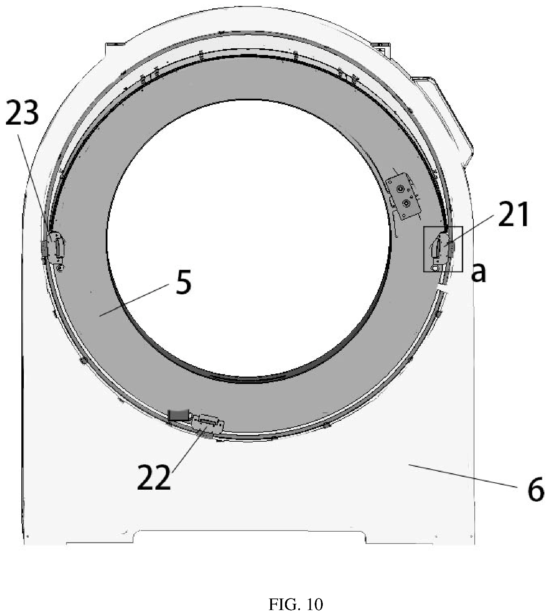

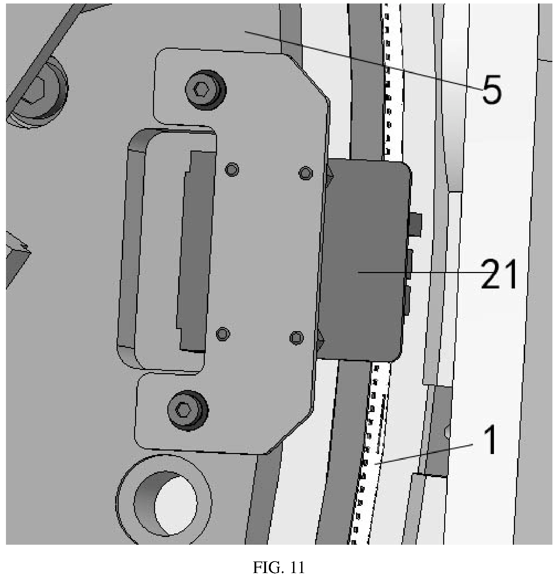

Based on the above-mentioned slip ring encoder and encoding method, the present disclosure further provides a CT device. The CT device will be described in detail below with reference to FIG. 10 to FIG. 11.

As shown in FIG. 10 to FIG. 11, FIG. 11 is a partial enlarged view of part a in FIG. 10. A CT device according to the present embodiment includes the encoder according to the above-mentioned embodiments. An encoding strip 1 and a slip ring 5 are mounted on a CT device body 6, and signal acquisition boards 21-23 are distributed on the slip ring 5. In the present embodiment, the encoding strip 1 remains stationary when the slip ring 5 rotates. For the signal acquisition boards 21-23, adjacent signal acquisition boards are arranged at a certain distance on one side of the encoding strip, and each signal acquisition board is configured to generate a first acquisition signal according to light emitted from the other side of the encoding strip and passing through the plurality of first-type apertures when rotating relative to the encoding strip. A plurality of long apertures may be arranged on the encoding strip, and one pressing piece may be mounted in each long aperture to fix the encoding strip to the CT device body. The pressing piece has a bent portion to press the encoding strip and prevent deformation of the encoding strip.

The above detailed description has illustrated numerous embodiments of the slip ring encoder and the encoding method thereof by using schematic diagrams, flowcharts, and/or examples. In the case that such schematic diagrams, flowcharts and/or examples include one or more functions and/or operations, those skilled in the art should understand that each function and/or operation in such diagrams, flowcharts or examples may be realized individually and/or jointly by various structures, hardware, software, firmware or any combination thereof in essence. In an embodiment, several parts of the subject matter described in embodiments of the present disclosure may be implemented by an application specific integrated circuit (ASIC), a field programmable gate array (FPGA), a digital signal processor (DSP), or other integrated formats. However, those skilled in the art should recognize that some aspects of embodiments disclosed herein in whole or in part may be equally implemented in an integrated circuit, implemented as one or more computer programs running on one or more computers (for example, implemented as one or more programs running on one or more computer systems), implemented as one or more computer programs running on one or more processors (for example, implemented as one or more computer programs running on one or more microprocessors), implemented as firmware, or substantially implemented as any combination of the above, those skilled in the art will have the ability to design a circuit and/or write software and/or firmware codes according to the present disclosure. In addition, those skilled in the art will recognize that the mechanism of the subject matter of the present disclosure may be distributed as a variety of forms of program products, and the exemplary embodiments of the subject matter of the present disclosure are applicable regardless of the specific type of signal carrier media actually used to perform distribution. Examples of signal carrier media include but are not limited to: recordable media, such as floppy disk, hard disk drive, compact disk (CD), digital versatile disk (DVD), digital tape, computer memory, etc.; and transmission type media, such as digital and/or analog communication media (e.g., an optical fiber cable, a waveguide, a wired communication link, a wireless communication link, etc.).

Embodiments of the present disclosure have been described above. However, these embodiments are for illustrative purposes only, and are not intended to limit the scope of the present disclosure. Although the various embodiments are described above separately, this does not mean that the measures in the various embodiments may not be advantageously used in combination. The scope of the present disclosure is defined by the appended claims and their equivalents. Without departing from the scope of the present disclosure, those skilled in the art may make various substitutions and modifications, and these substitutions and modifications should all fall within the scope of the present disclosure.

## Claims

1. A slip ring encoder, comprising:
an encoding strip (1) having a plurality of first-type apertures regularly arranged;
a plurality of mutually independent signal acquisition boards (21-23) arranged on one side of the encoding strip (1), wherein each of the signal acquisition boards comprises a first signal sensor configured to, when rotating relative to the encoding strip (1), generate a first acquisition signal according to light emitted from the other side of the encoding strip (1) and passing through the plurality of first-type apertures; and
a signal processing board (4) electrically connected to the plurality of signal acquisition boards (21-23) and configured to generate a first encoding signal based on at least one of a plurality of first acquisition signals.

2. The slip ring encoder according to claim 1, wherein the plurality of signal acquisition boards (21-23) are arranged on one side of the encoding strip (1), and the plurality of signal acquisition boards (21-23) are configured to generate the plurality of first acquisition signals having a substantially same phase.

3. The slip ring encoder according to claim 1 or 2, wherein the plurality of signal acquisition boards (21-23) are arranged separately from the signal processing board (4).

4. The slip ring encoder according to any one of the preceding claims, wherein generating the first encoding signal based on at least one of the plurality of first acquisition signals comprises:
the signal processing board (4) being configured to select one of the first acquisition signals to generate the first encoding signal,
wherein, in a case that the selected first acquisition signal is lost, another first acquisition signal is selected to generate the first encoding signal.

5. The slip ring encoder according to any one of the preceding claims, wherein adjacent signal acquisition boards (21-23) are arranged on one side of the encoding strip (1) at a central angle interval of more than 1°.

6. The slip ring encoder according to any one of the preceding claims, wherein each of the signal acquisition boards (21-23) further comprises a second signal sensor configured to, when rotating relative to the encoding strip (1), generate a second acquisition signal according to light emitted from the other side of the encoding strip (1) and passing through the plurality of first-type apertures;
the first acquisition signal and the second acquisition signal generated by each of the signal acquisition boards (21-23) have a preset phase difference, based on a relative position difference between the first signal sensor, the second signal sensor and any one of the plurality of first-type apertures;
the signal processing board (4) is further configured to generate a second encoding signal based on at least one of a plurality of second acquisition signals; and
the first encoding signal and the second encoding signal are configured to determine a rotation direction of a slip ring.

7. The slip ring encoder according to claim 6, wherein the preset phase difference between the first acquisition signal and the second acquisition signal generated by each of the signal acquisition boards (21-23) is between 1/4 and 3/4 of a signal period.

8. The slip ring encoder according to any one of the preceding claims, wherein a second-type aperture is provided on a circumference of the encoding strip (1), and a radius of the circumference for the second-type aperture is different from a radius of a circumference for the first-type aperture; each of the signal acquisition boards (21-23) further comprises a third signal sensor configured to, when rotating relative to the encoding strip (1), generate a third acquisition signal according to light emitted from the other side of the encoding strip (1) and passing through the second-type aperture; and
the signal processing board (4) is further configured to generate a third encoding signal based on at least one of a plurality of third acquisition signals, and the third encoding signal is configured to determine a number of rotations of a slip ring.

9. An encoding method for a slip ring encoder, applied to the slip ring encoder according to any one of claims 1 to 8, wherein the method comprises:
when a plurality of first signal sensors of a plurality of signal acquisition boards (21-23) rotate relative to an encoding strip (1), generating a plurality of first acquisition signals based on light emitted from the other side of the encoding strip (1) and passing through a plurality of first-type apertures; and
generating a first encoding signal based on at least one of the plurality of first acquisition signals.

10. The encoding method according to claim 9, further comprising:
detecting whether the first acquisition signal is lost; and
indicating an abnormal position of the encoding strip (1) based on a loss position of the first acquisition signal.

11. The encoding method according to claim 9 or 10, wherein the generating a first encoding signal based on at least one of the plurality of first acquisition signals comprises:
selecting one of the first acquisition signals to generate the first encoding signal,
wherein, in a case that the selected first acquisition signal is lost, another first acquisition signal is selected to generate the first encoding signal.

12. The encoding method according to one of the claims 9 to 11, further comprising:
when a plurality of second signal sensors of the plurality of signal acquisition boards (21-23) rotate relative to the encoding strip (1), generating a plurality of second acquisition signals according to light emitted from the other side of the encoding strip (1) and passing through the plurality of first-type apertures;
generating a second encoding signal based on at least one of the plurality of second acquisition signals; and
determining a rotation direction of a slip ring based on the first encoding signal and the second encoding signal,
wherein each of the signal acquisition boards (21-23) further comprises the second signal sensor,
the first acquisition signal and the second acquisition signal generated by each of the signal acquisition boards (21-23) have a preset phase difference, based on a relative position difference between the first signal sensor, the second signal sensor and any one of the plurality of first-type apertures.

13. The encoding method according to one of the claims 9 to 12, further comprising:
when a plurality of third signal sensors of the plurality of signal acquisition boards (21-23) rotate relative to the encoding strip (1), generating a plurality of third acquisition signals according to light emitted from the other side of the encoding strip (1) and passing through a second-type aperture;
generating a third encoding signal based on at least one of the plurality of third acquisition signals; and
determining a number of rotations of a slip ring based on the third encoding signal,
wherein the second-type aperture is provided on a circumference of the encoding strip (1), a radius of the circumference for the second-type aperture is different from a radius of a circumference for the first-type aperture, and each of the signal acquisition boards (21-23) further comprises the third signal sensor.

14. The encoding method according to claim 13, wherein the determining a number of rotations of a slip ring based on the third encoding signal comprises:
accumulating the first encoding signal, and
incrementing the number of rotations of the slip ring by one based on the third encoding signal and an accumulated count value of the first encoding signal reaching a preset value.

15. A CT device, comprising the slip ring encoder according to any one of claims 1 to 8.
